# EUROPEAN PATENT APPLICATION

(11) **EP 4 302 854 A2**
(43) Date of publication of application: **10.01.2024**
(21) Application number: 23179944.6
(22) Date of filing: 19.06.2023
(51) Int. Cl.: B01D 11/02, A23L 33/105, A61K 8/00, A61K 8/73, A61K 8/97, A61K 36/752

(54) **PROCESS OF FRACTIONATION AND RECOVERY OF FLAVONOIDS, TERPENES AND PREBIOTIC FIBER CONTAINING POLYSACCHARIDES FROM PLANT MATERIAL, USE OF PLANT MATERIAL, AND FLAVONOIDS, TERPENES AND PREBIOTICAL FIBER CONTAINING POLYSACCHARIDES**

(30) Priority: 08.07.2022 BR 102022013691
(71) Applicant: Fiber Citrus Indústria E Comérció Ltda., CEP 01451-010 São Paulo SP (BR)
(72) Inventor: DA SILVA SOARES, FILIPE, CIDADE DE SÃO JOSÉ DO RIO PRETO (BR); JOSÉ GUERRA, FRANCISCO, CIDADE DE MATÃO (BR); RODRIGUES LOPES, OTHNIEL, SÃO PAULO (BR)
(74) Representative: Lavoix

(57) **Abstract**

The present invention relates to a process for fractionation and total recovery of flavonoids, terpenes and polysaccharides from the bark of a vegetable material, such as citrus peel, without generating effluents. The present invention also refers to flavonoids, terpenes and polysaccharides obtained by the process and their use in various applications in the food, cosmetic, pharmaceutical or chemical areas. The process developed by the present inventors allows the total recovery of the flavonoids, terpenes and polysaccharides components without the use of extreme pH and temperature conditions and without generating effluents, providing a clean and sustainable process, since citrus peel is used as a material for starting point and comprises steps of acid synthesis by means of a metal ion sequestration mechanism.

## Description

### INVENTION FIELD

The present invention relates to a fractionation process and total recovery of flavonoids, terpenes and prebiotic fiber containing polysaccharides from a plant material, such as citrus peel.

Particularly, the present process consists of a continuous and integrated process capable of fractionating and recovering different products, such as flavonoids, terpenes and prebiotic fiber containing polysaccharides, with the use of all plant material, without the use of extreme pH conditions and temperature and without the generation of effluents, providing a clean and sustainable process.

The waste materials generated consist of a product rich in nitrogen, phosphorus and potassium that can be later safely used as fertilizer or in vegetable water that can safely return to nature or be fed back into the process. The present invention also refers to flavonoids, terpenes and prebiotic fiber products containing polysaccharides obtained by the process and their use in various applications in the food, cosmetic, pharmaceutical or chemical areas.

### BACKGROUND OF THE INVENTION

Flavonoids, terpenes and polysaccharides are compounds normally used in the pharmaceutical, cosmetic or food field and have specific functions depending on their application.

Flavonoids refer to a class of phenolic compounds and are found in the plant kingdom, having important functions for the development of a plant. Flavonoids are also important in the performance of pharmacological activities in the human body, and may have antioxidant, antiviral, antiinflammatory, antitumor properties, among others, which is a component that has aroused interest in the pharmaceutical market. Examples of flavonoids that have pharmacological activities are acacetin, amentoflavone, butein, hesperidin, quercetin, rutin, etc.

Terpenes or terpenoids are substances naturally produced by a plant material and, due to their fragrance, are normally used as components of essential oils, but also have pharmacological properties, being able to act as anticancer, bactericidal, fungicidal, etc. Terpenes can also be used in food preservation, as a cosmetic ingredient, in organic agriculture as an insecticide, as flavoring agents and in household cleaning. The most common terpene sourced from citrus fruits is limonene.

Polysaccharides are natural polymers and may consist of a single or different types of monosaccharides. The main polysaccharide extracted from the cell wall of fruits belongs to the class of pectins. They are heteropolysaccharides consisting of a linear backbone of α-D-galacturonic acid and its O-methylated derivatives. They have several applications, such as food, cosmetics and pharmaceuticals, especially in the food industry (as a stabilizer and thickener). In the present invention, the polysaccharides are marketed in the form of prebiotic fiber due to their functional characteristics.

Flavonoids, terpenes and fibers containing polysaccharides can be obtained by conventional processes by extracting the compound of interest at pH below 2.5 by acid hydrolysis or by enzymatic action, said processes require activation by temperature rises greater than 40 °C.

Conventional processes, in addition to using steps and parameters involving more extreme reaction conditions of pH and temperature, also lead to the generation of liquid and solid effluents.

Another disadvantage of conventional processes is that it is possible to obtain only one type of compound, usually pectin (which contains mostly flavonoids), and terpenes and polysaccharides are discarded in the process, that is, there is no process that allows the total recovery of these 3 compounds.

The prior art document BR 112016012973-3 describes a process that aims to extract only pectin from a starting material, involving only extraction steps. Said process comprises a first step a) of extraction with acid using an acid solution with a pH between 1.5 to 2.5, optionally a second step b) in which the first residue is subjected to one to three washes and a third step c ) comprising a third extraction.

The inventors of this application verified that the processes available in the prior art do not allow the fractionation and total recovery of flavonoids, terpenes and prebiotic fiber containing polysaccharides from the same plant material and in a continuous process.

The process developed by the present inventors allows the total recovery of the flavonoid components, terpenes and prebiotic fiber containing polysaccharides without the use of extreme conditions of pH and temperature and without generating effluents, providing a clean and sustainable process, since it is uses citrus peel as starting material.

Additionally, the process of the present invention allows a high yield in terms of using the citrus peel since it consists of a metal sequestration mechanism, rather than the use of a conventional extraction mechanism.

Thus, there is a visible need to develop new processes for obtaining products from plant material that allow for greater yields and that are integrated in order to allow the recovery of the largest number of compounds of interest to the market and that are sustainable way to minimize or even eliminate the generation of effluents.

### SUMMARY OF THE INVENTION

Thus, an objective of the present invention is to provide a fractionation process and total recovery of flavonoids, terpenes and prebiotic fiber containing polysaccharides from citrus plant material.

A second objective of the invention is to obtain flavonoids, terpenes and prebiotic fiber containing polysaccharides obtained by the process of the present invention and their use in various applications in the food, cosmetic, pharmaceutical or chemical areas.

A third objective of the present invention is to provide a process that uses mild process conditions and does not generate effluents, in which the liquid generated is used as an organic fertilizer and the plant water generated is reused in the process or returned to nature in a clean way .

Basically, the present invention relates to a process comprising the main steps:
1) Adjust the granulometry of the plant material (1');
2) Place the ground plant material in a reactor 1 (3) and add a complexing compound (16) and a solvent (35);
3) Add an acidic compound (17) to the mixture obtained in step 2) and separate the mixture to obtain a liquid phase containing flavonoids and terpenes and a first retained solid mass;
4) Insert the first retained solid mass obtained into a reactor 2 (19) and add the complexing compound (16), an acid compound (17), a solvent (35) and separate the mixture obtained to obtain a liquid phase containing terpenes and a second retained solid mass;
5) Insert the second retained solid mass obtained into a reactor 3 (23) and add a solvent (35); and
6) Neutralize the mixture obtained in step (5) by adding a neutralizing compound (18) and recover the functional fiber containing polysaccharides (33').

### BRIEF DESCRIPTION OF THE FIGURES

The objectives, technical effects and advantages of the present invention will be apparent to those skilled in the art from the detailed description below, which makes reference to the attached figures, which illustrate an exemplary, but not limiting, embodiment of the process of the present invention.

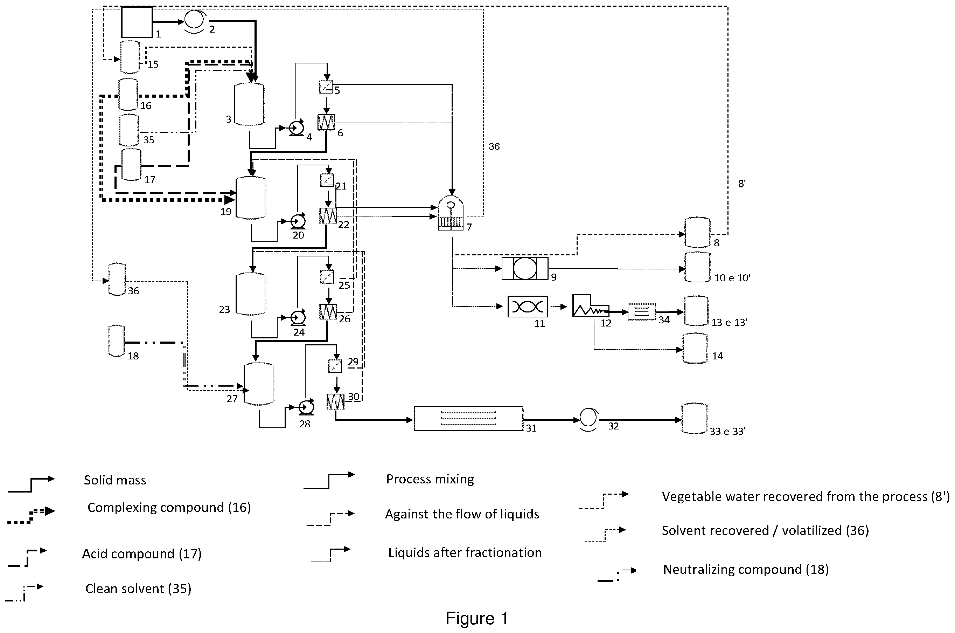

Figure 1 shows a schematic flowchart of the fractionation and recovery process of the present invention, containing the following steps and components:
   - Receipt silo (1) of raw material or plant material (1');
      - Mill (2);
      - Reactor 1 (3):
      - Transfer pump 1 (4);
      - Sieve 1 (5):
      - Press 1 (6);
      - Concentrator/distillator (7);
      - Water tank 1 (8) and vegetable water (8');
      - Capitation of terpenes (9);
      - Storage of terpenes (10) and terpenes (10');
      - Crystallization of flavonoids (11);
      - Ultrafiltration system (12);
      - Flavonoids Storage Tank (13) and flavonoids (13');
      - Organic fertilizer storage tank (14) and organic fertilizer (14');
      - Water tank 2, recovered from the process (15);
      - Complexing compound (16);
      - Acid compound (17);
      - Neutralizing compound (18);
      - Reactor 2 (18);
      - Transfer pump 2 (20);
      - Sieve 2 (21);
      - Press 2 (22);
      - Reactor 3 (23);
      - Transfer pump 3 (24);
      - Sieve 3 (25);
      - Press 3 (26);
      - Reactor 4 (27);
      - Transfer pump 4 (28);
      - Sieve 4 (29);
      - Press 4 (30);
      - Drying system 1 (31);
      - Mill 2 (32);
      - Storage of fiber (33) and fiber containing polysaccharides (33');
      - Drying system 2 (34);
      - Clean Solvent (35); and
      - Solvent recovered/volatilized (36).

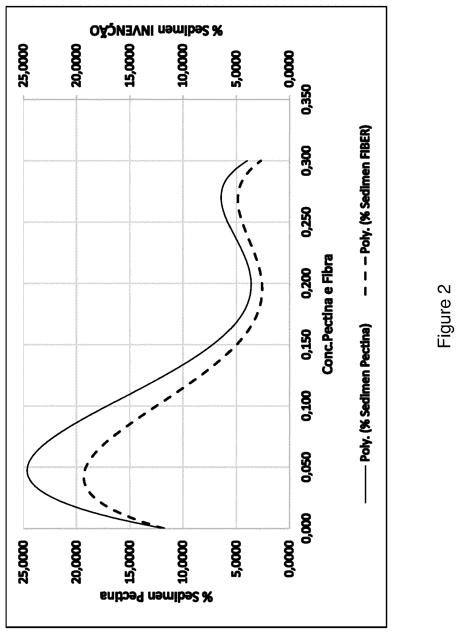

Figure 2 shows a graph containing a polynomial curve of the ratio of % sediment versus pectin and fiber concentration in the milk protein stability test.

### DETAILED DESCRIPTION OF THE INVENTION

The present invention relates to a fractionation process and total recovery of flavonoids, terpenes and prebiotic fiber containing polysaccharides from a citrus plant material.

During the ripening cycle of a citrus fruit, there is the consumption of some compounds, such as terpenes, which result in the characteristic citrus aroma. Flavonoids are natural pigments that play a key role in protecting the fruit against oxidizing agents and ultraviolet rays, while structural carbohydrates such as polysaccharides are also consumed by the fruit during the ripening process, which make the vegetable skin less rigid.

The process of the present invention contemplates the fractionation and total recovery of the compounds mentioned above, converting them into segregated products. The possibility of recovering these three compounds in the same production line is only possible due to a means of complexometric reaction developed by the inventors of the present invention, in which there is a sequestration of metallic ions.

The raw material used in the process is a plant material, or, particularly, a vegetable peel comprising the peel of a citrus fruit. The citrus fruits that can be used in the invention are orange, Tahiti lemon, Sicilian lemon, tangerine, lime, grapefruit, mandarin orange, pomelos and mixtures thereof. The citrus peel used comprises any of its parts, including the core, frits, albedo, flavedo or pulp.

The process of the present invention basically consists of receiving plant material based on citrus peel, which is a by-product generated from a citrus juice extraction factory. The citrus peel undergoes a granulometry adjustment and is subsequently sent to a No. 1 reactor where a complexing compound and a solvent are added to promote the synthesis of metal sequestration. Then, an acidic compound is added in reactor nº 1 in order to allow the recovery of flavonoids as well as organic fertilizer based on NPK (nitrogen, phosphorus and potassium).

Subsequently, the solid mixture is sent to reactor No. 2, where a solvent, a complexing compound and acidic compound are added to release terpenes and finally, the solid mixture is sent to reactor No. 3, where it occurs the acid synthesis to release the polysaccharides and finally, the solid mixture is sent to a reactor nº 4, where the neutralization and recovery of the prebiotic containing polysaccharides takes place.

As a result, the process of the invention does not generate effluents that are harmful to the environment, since the process eliminates only two types of liquid waste, the first being vegetable water, which can be safely disposed of or reused in the process and the second being a concentrate of nitrogen, phosphorus and potassium, which can be reused as an organic fertilizer.

An advantage of the present invention is that the process allows the recovery of 80 to 95% of the solid mass, said solid mass containing flavonoids and with functional capabilities.

Another advantage of the present invention lies in the fact that the generation of effluents is minimized and completely eliminated since the process of the invention allows recovering from 80 to 95% of the vegetable water contained in the wet raw material, and the vegetable water can be safely discarded or reused in the process.

Still, an additional advantage of the invention is to provide an integrated process allowing the fractionation and recovery of more than 70% of the flavonoids that are available in the citrus fruit, with a yield greater than 1% wet basis and 6% dry basis.

The main steps that make up the process of the present invention are:
1) Adjust the granulometry of the plant material (1') in a mill (1);
2) Place the ground plant material in a reactor 1 (3) and add a complexing compound (16) and a solvent (35);
3) Add an acidic compound (17) to the mixture obtained in step 2);
   3.1- optionally, the water tank 2 (15), which is recovered from the process, can be inserted into the mixture obtained in step 3);
   3.2- separate the mixture obtained in step 3) to obtain a liquid phase containing flavonoids and terpenes and a first retained solid mass;
   3.3- submit the liquid phase containing flavonoids and terpenes to a concentrator/distiller (7) to separate the flavonoids (13'), vegetable water (8') and NPK fertilizer (14') and the volatilized solvent (36), where said volatilized solvent returns to the process in reactor 4 (27);
4) Insert the first retained solid mass obtained in step 3.2 into a reactor 2 (19) and add the complexing compound (16), an acid compound (17), a solvent (35) and, optionally, at least one solvent recovered from the process (25 or 26);
   4.1- separate the mixture obtained in step 4) to obtain a liquid phase containing terpenes and a second retained solid mass;
   4.2- submit the liquid phase containing terpenes to a concentrator/distillator (7) generating terpenes (10');
5) Insert the second retained solid mass obtained from step 4.1 into a reactor 3 (23) and add a solvent (35) and, optionally, at least one solvent recovered from the process (29 or 30);
   5.1- separate the mixture obtained in step 5) to obtain a residual liquid phase and a third retained solid mass; and
6) Forward the third retained solid mass obtained in step 5.1 into a reactor 4 (27) and neutralize the mixture by adding a neutralizing compound (18) and the volatilized solvent (36) from the concentrator/distiller (7) and recover the fiber functional polysaccharide-containing (33').

Each of the six main steps that make up the invention process are detailed below.

### TERMS

O The use of the term "one" in this descriptive report does not indicate a limited quantity, but the existence of at least (at least) one of the elements/components/items listed.

The use of the term "or" indicates any or all of the elements/components/items listed.

The use of the term "comprehend", "endowed", "provided" or a similar term indicates that the element/component/item listed in front of said term forms part of the invention, but does not exclude other elements/components/items not listed.

As used in this specification, the expression "at least one" means one or more and therefore includes individual components, as well as mixtures of 1, 2, 3 or more components and/or combinations.

Except in the operational examples, or where otherwise indicated, all numbers expressing reaction conditions are to be understood as modified in all cases by the term "about", meaning within +/- 10% of the indicated number.

As used in this document, all ranges provided must include all specified ranges within and combinations of subranges between the ranges provided. Thus, a range of 1 to 5, for example, specifically includes 1, 2, 3, 4 and 5, as well as subranges such as 2 to 5, 3 to 5, 2 to 3, 2 to 4, 1 to 4, etc. All ranges and values disclosed here are inclusive and combinable. For example, any value or point described here that is within an interval described here can serve as a minimum or maximum value for deriving a subinterval, and so on.

The processes of the present invention may comprise, consist of, or consist essentially of the essential elements and limitations of the invention described herein, as well as any additional or optional ingredients, components or limitations described or useful herein.

According to the present invention, the term "vegetable water" refers to the liquid portion that is removed in the concentrator / distiller from the initial solid mass and consists basically of water. The vegetable water generated in the present invention is usually reused in the process and fed back into the water tank 2 (15) or, when in excess, it can be safely discarded in nature, as it does not contain toxic products in its composition.

The term "complexing compound" or "chelating" refers to a compound that is capable of forming a chelate, that is, that is capable of forming complexes with the cations present in the plant material.

The term "solvent (35)" consists of an alcohol selected from, but not limited to: ethyl alcohol, propyl alcohol, butyl alcohol, or mixtures thereof. Preferably, solvent (35) is ethyl alcohol. The solvent is fed into the reactor in its clean form, i.e. containing the solvent in its pure form. The solvent (35) also consists of the solvent recovered from the process, which contains at least 80% of its initial purity, even more preferably at least 90% of its initial purity, and may also contain other process components such as the complexing compound or a acidic compound.

The term "recovered or volatilized solvent (36)" consists of the liquid recovered from the concentrator/distiller (7) and is reused in the process and contains basically water and solvent residues (35). The solvent is recovered to at least 80%, more preferably 90% of its initial purity.

### STAGE 1: PLANT MATERIAL GRANULOMETRY ADJUSTMENT

This step consists of adjusting the granulometry of the plant material fed into the process. Adjusting the granulometry is important so that the entire surface of the plant matter comes into contact during the reaction, making the sequestration of ions more efficient.

For this adjustment, a silo (1) is initially used to receive the raw material in order to receive and store the citrus peel from the citrus juice extraction process. Citrus peels, such as orange peel, after juice extraction, become waste for juice factories. Such citrus peels, which would usually be discarded, will serve as raw material for the present process for the reuse of products contained in the peel.

After storage in the silo (1), the bark is sent to a mill (2), preferably a knife mill. At this stage, the citrus fruits are crushed until they reach granulometry in the range of 2 mm to 20 mm.

In one embodiment, the plant material is ground to a diameter ranging from 5mm to 15mm, even more preferably from 5mm to 10mm.

This granulometry adjustment step is important in the process to allow the entire surface of the plant matter to come into contact with the reaction medium, making the sequestration of ions more efficient.

### STEP 2: MIXING PLANT MATERIAL WITH A COMPLEXING COMPOUND AND A SOLVENT TO PROMOTE METAL SEQUESTRATION SYNTHESIS

After the granulometry step, the ground plant material is inserted into Reactor 1 (3), where the addition of a complexing compound (16) and a solvent (35) occurs.

The purpose of this step 2) is to sequester the metals present in the plant material, detaching them from the cell structure as a result of removing natural sugars when they lose their binding strength.

The complexing compound (16) added in this step is selected from, but not limited to: EDTA (ethylenediaminetetraacetic acid), DTPA (diethylenetriamine pentaacetic acid), NTA (nitrile triacetate) and mixtures thereof. Preferably, the chelating compound (16) is EDTA.

The solvent (35) used in this step 2) is selected from, but not limited to: ethyl alcohol, propyl alcohol, butyl alcohol, or mixtures thereof. Preferably, solvent (35) is ethyl alcohol. The solvent is fed into the reactor in its clean form, i.e. containing the solvent in its pure form.

In another embodiment, the solvent (35) consists of the solvent recovered from the process, which contains at least 80% of its initial purity, even more preferably at least 90% of its initial purity, and may also contain other components of the process such as as the complexing compound or an acidic compound.

In a preferred embodiment, Reactor 1 (3) consists of mixing the ground plant material, the complexing compound (17) of the solvent (35) and, optionally, the plant water (8') from the water tank 2 recovered from the process (15).

The complexing compound (17) is inserted in this step at a concentration ranging from 5% to 40%, preferably at a concentration of 10 to 30% by weight, even more preferably 15%.

The concentration of the solvent (35) in step 2) ranges from 40% to 50% by weight, preferably 45% by weight.

Particularly, the reactor 1 (3) of step 2) comprises the mixture of a ground plant material (1') obtained from step 1), a complexing compound (16) and a solvent compound (35), in which such mixing takes place at a pH ranging from 3.0 to 4.5, at a temperature ranging from 20 to 35°C and under stirring from 30 to 42 rpm. Such a reaction occurs at a continuous flow ranging from 20 to 60 minutes. The temperature, mixing time and agitation speed may vary according to the citrus variety and region due to the nutrients absorbed from the soil by the tree.

### STAGE 3: ADDITION OF ACID COMPOUND AND OBTAINING FLAVONOIDS AND ORGANIC FERTILIZER

After mixing a complexing compound (16) and a solvent (35) to the ground plant material in the reactor 1(3), an acidic compound (17) is added to the reaction medium.

The addition of an acidic compound allows the solubility of flavonoids present in the cell wall of the material to be facilitated.

Basically, step 3) consists of the following steps and sub-steps:
Step 3): Add an acidic compound (17) in reactor 1 (3) to the mixture obtained in step (2);
Step 3): Add an acidic compound (17) in reactor 1 (3) to the mixture obtained in step (2);
3.1- optionally, the vegetable water (8') that is recovered from the process, coming from the water tank 2 (15), can be inserted into the mixture obtained in step 3);
3.2- separate the mixture obtained in step (3) to obtain a liquid phase containing flavonoids and terpenes and a first retained solid mass;
3.3- submit the liquid phase containing flavonoids and terpenes to a concentrator/distiller (7) to separate the flavonoids (13'), vegetable water (8') and NPK fertilizer (14') and the volatilized solvent (36), where said volatilized solvent returns to the process in reactor 4 (27);

The acid compound (17) that can be used in step 3) is selected from: nitric acid, hydrochloric acid, sulfuric acid, acetic acid or mixtures thereof. Preferably, the acidic compound is a nitric acid.

In one embodiment, the acid is added in its pure form or dissolved in an organic solvent, such as water.

The amount of acid driven is less than 10%, preferably less than 5% of the total volume of the mixture, more preferably less than 2% of the total volume of the mixture, even more preferably between 0.1% to 5%, or even more preferably between 0.1% to 2%.

Particularly, the reactor 1 (3) of step 3) comprises a mixture of ground plant material (1'), a complexing compound (16), a solvent compound (35), and an acid compound (17) in that such mixing occurs at a pH ranging from 2.5 to 3.5, at a temperature ranging from 20 to 35°C and agitation from 30 to 42 rpm. Such a reaction occurs at a continuous flow ranging from 20 to 60 minutes. Temperature, mixing time and agitation speed may vary according to citrus variety and region.

After adding the acid solution, the mixture goes through the following sub-steps:
In sub-step 3.2 of separation, the mixture is drained using sieve 1 (5) and press 1 (6).

The sieve 1 (5) occurs through the action of gravity of all the components that were sequestered from the plant matter, which are separated in a closed mesh, directing the retained solid mass to the press 1 (6) and the product liquid is directed to the concentrator/distiller (7).

The press step 1 (6) consists of receiving the solid mass from the sieve 1 (5) and reducing the humidity of the solid mass by up to 75%, directing said solid mass to reactor 2 (19). The liquid product resulting from this step is further directed to the concentrator/distillator (7).

Sub-step 3.3 consists of the separation of the liquid phase in the concentrator/distillator (7). This step consists of concentrating the liquid product obtained on sieve 1 (5) at controlled temperatures of 70 to 92 °C to a concentration of 20 to 65° brix. The volatilized solvent (36) is recovered in distillation columns to return to the process in reactor 4 (27). The vegetable water (8') removed from the initial solid mass is directed to the water tank 1 (8) which supplies the water tank 2 (15) feeding the process or returning to nature.

The concentrate containing flavonoids obtained in the concentrator/distiller (7) of step 3.3 is sent to a crystallizer (11) where the flavonoid crystallization process occurs. In this sub-step, a neutralizing compound (18) is added to the concentrate so that it reaches a pH range between 8 to 11, allowing the crystallization of flavonoids (preferably hespedins) at a temperature ranging from 15 to 30ºC for a time ranging from 20 to 300 minutes and no mechanical power.

The neutralizing compound (18) used in sub-step 3.3 is preferably an alkaline compound, even more preferably potassium hydroxide.

The concentrate obtained after the crystallization step (sub step 3.3) is further directed to a tangential flow ultra-filtration system (12) where the separation of a liquid that is rich in metals, sugars and nutrients occurs, generating a product characterized as an organic fertilizer rich in nitrogen, phosphorus and potassium - NPK (14').

The solid retained in the ultrafiltration membranes is directed to the drying system (34), controlled at a temperature of 50 to 65°C and goes to the storage of flavonoids (13), obtaining the flavonoids (13').

Storage tanks can be used in the process for storing the products obtained, for example, a tank for storing flavonoids (13), a tank for storing organic fertilizer (14), water tank 1 (8) to capture the vegetable water (8') removed from the initial solid mass and a water tank 2 (15) for storing the vegetable water (8') recovered in the process.

### STAGE 4: ADDITION OF SOLVENT, COMPLEXING COMPOUND AND ACID COMPOUND FOR THE RELEASE OF

**TERPENES**The first retained mass that is obtained in the separation step (sub step 3.2) is directed to a reactor 2 (19).

Particularly, this step comprises mixing the mass from press 1 (6) with the solvent (35), the complexing compound (16), the acid compound (17) and the addition of the liquids recovered from the process, which come from sieve 3 (25) and press 3 (26).

Optionally, adjustments in the concentration of alcohol, complexing and acid reagents can be performed.

The purpose of this step is to solubilize the terpenes present in the solution and allow the subsequent removal of these terpenes.

Step 4 consists of the following sub-steps:
Step 4): Insert the first retained solid mass obtained in step 3.2 into a reactor 2 (19) and add the complexing compound (16), an acid compound (17), a solvent (35) and at least one solvent recovered from the process , such as the liquid from the sieve 3 (25) or the liquid from the press 3 (26);
4.1- separate the mixture obtained in step 4) to obtain a liquid phase containing terpenes and a second retained solid mass;
4.2- submit the liquid phase containing terpenes to a concentrator/distillator (7) generating terpenes (10').

In a preferred embodiment, in this step 4, the solvent (35) is selected from, but not limited to: ethyl alcohol, propyl alcohol, butyl alcohol, or mixtures thereof. Preferably, solvent (35) is ethyl alcohol.

The solvent (35) is present in a concentration ranging from 40 to 60%, preferably 50%.

In another preferred embodiment, the complexing compound of step 4 is selected from, but not limited to: EDTA (ethylenediaminetetraacetic acid), DTPA (diethylenetriamine pentaacetic acid), NTA (nitrile triacetate) and mixtures thereof. Preferably, the complexing compound (16) is EDTA.

The complexing compound (16) is at a concentration ranging from 5% to 40% by weight, preferably at a concentration of 10 to 30% by weight, even more preferably 15%.

The acid compound (17) that can be used in step 4) is selected from: nitric acid, hydrochloric acid, sulfuric acid, acetic acid or mixtures thereof. Preferably, the acidic compound is a nitric acid.

In one embodiment, the acid is added in its pure form or dissolved in a solvent, such as water.

The acid compound (17) of step 4) is in a concentration that does not exceed 5% of the total mass of the mixture, preferably not exceeding 2%, even more preferably, the acid compound (17) is in a range of 0.1 to 2%.

Particularly, the reactor 2 (19) of step 4) comprises the mixture of the first retained solid mass obtained from press 1 (6), with a complexing compound (16), a solvent compound (35), and an acid compound (17) as well as liquids from sieve 3 (25) and press 3 (26), so that such mixture reaches a pH range ranging from 2.5 to 4.5, at a temperature ranging from 35 to 55ºC and agitation from 30 to 42 rpm. Such a reaction occurs at a continuous flow ranging from 30 to 60 minutes. Temperature, mixing time and agitation speed may vary according to citrus variety and region.

The pH adjustments in the reaction mixture are made with the complexing agent itself (16) and/or acidic compound (17).

In sub step 4.1 of separation, the mixture is drained using sieve 2 (21) and press 2 (22).

Optionally, a transfer pump (20) can be used in order to unload the reactor 2 (19) to the sieve 2 (21).

The sieve 2 (21) occurs through the action of gravity of all components that were sequestered from the plant matter, which are separated in a closed mesh, directing the retained solid mass to the press 2 (21) and the product liquid is directed to the concentrator/distiller (7).

The press step 2 (22) consists of receiving the solid mass from the sieve 2 (21) and reducing the humidity of the solid mass by up to 60%, directing said solid mass to the reactor 3 (23). The liquid product resulting from this step is further directed to the concentrator/distillator (7).

In sub-step 4.2 the liquid phase containing terpenes is subjected to a concentrator/distillator (7). In a preferred embodiment, the liquid phase containing terpenes is additionally directed to a distillation column (9), where the uptake of the terpenes (10') takes place at a controlled temperature of 40 to 95°C. Fractionation takes place according to the volatility differential and is then sent to a terpene storage tank (10).

The vegetable water (8') that was generated in this step is directed to the water tank 1 (8) which supplies the water tank 2 (15) feeding the process or returning to nature.

### STEP 5: ACID SYNTHESIS TO RELEASE POLYSACCHARIDES

One of the main objectives of step 5 is to remove residuals from previous processes, in which the mass from press 2 (22) is mixed in a reactor 3 (23) with the solvent (35) and the addition of the liquids eventually recovered from the process, which come from sieve 4 (29) and press 4 (30).

The solvent (35) used in this step 5) is selected from, but not limited to: ethyl alcohol, propyl alcohol, butyl alcohol, or mixtures thereof. Preferably, solvent (35) is ethyl alcohol.

This step occurs without raising the temperature between a period ranging from 10 to 30 min under stirring ranging from 30 to 42 rpm. The concentration of the solvent (35) in this step 5 can vary from 40 to 70%. In a preferred embodiment, in this step 5, the solvent is present in a preferred concentration of 50 to 60%.

The mixing time and stirring speed may vary according to the citrus variety and region.

Optionally, adjustments in the concentration of the solvent (35) can be performed.

Step 5 comprises the following sub-steps:
Step 5) - insert the second retained solid mass obtained from step 4.1 into a reactor 3 (23) and add a solvent (35) and at least one solvent recovered from the process (29 or 30);
5.1- separate the mixture obtained in step 5) to obtain a residual liquid phase and a third retained solid mass.

In sub-step 5.1 of separation, the mixture is drained using sieve 3 (25) and press 3 (26).

Optionally, a transfer pump (24) can be used in order to unload the reactor 3 (12) to the sieve 3 (25).

The sieve 3 (25) occurs through the action of gravity of all the residual components that have been sequestered, which are separated in a closed mesh, directing the retained solid mass to the press 3 (26) and the liquid product is directed to reactor 2 (19) in a counterflow system.

The press step 3 (26) consists of receiving the solid mass from the sieve 3 (25) and reducing the moisture of the solid mass by up to 50%, said solid mass being directed to the reactor 4 (27). The liquid product resulting from this step is further directed to reactor 2 (19) in a counterflow system.

### STAGE 6: NEUTRALIZATION AND RECOVERY OF PREBIOTICS POLYSACCHARIDES

The objective of the step is to stabilize the mass composed of cellulose, hemicellulose, lignins, among other carboxylic groups such as pectins, forming a prebiotic fiber with different functionalities.

Step 6 consists of mixing in reactor 4 (27) the mass from press 3 (26) with the solvent (35) that was recovered from the concentrator/distillator (7) and adding a neutralizing compound (18) .

The mixture from step 6 has the pH adjusted to ranges ranging from 3.5 to 6.5 with an acid or base. This step occurs without temperature increase between 10-30 min and agitation varies between 30 to 42 rpm. Mixing time and agitation speed may vary according to citrus variety and region.

The acids that can be used in the neutralization are selected from: nitric, hydrochloric, sulfuric, acetic acid, and mixtures thereof. Bases for neutralization are selected from potassium hydroxide, sodium hydroxide, and mixtures thereof. Preferred acids and bases are nitric acid and potassium hydroxide, respectively.

The concentration of solvent (35) in step 6 can vary between 50 to 80%, preferably 60 to 70%.

The retained mass is then subjected to the following sub-steps:
6.1- separate the mixture into a liquid phase and a resulting mass rich in polysaccharide fiber; and
6.2 - drying the resulting mass in a drying system 1 (31), followed by grinding in a mill 2 (32) and a drying system 2 (34) obtaining the prebiotic fiber containing polysaccharides (33').

In sub step 6.1 of separation, the mixture is drained using sieve 4 (29) and press 4 (30).

Optionally, a transfer pump (28) can be used in order to unload the reactor 4 (27) to the sieve 4 (29).

Sieve 4 (29) occurs through the action of gravity of the mixture, which are separated in a closed mesh, directing the retained solid mass to press 4 (30) and the liquid product is directed to reactor 3 (23 ) in a counterflow system.

The press step 4 (26) consists of receiving the solid mass from the sieve 4 (29) and reducing the moisture of the solid mass by up to 50%, said solid mass being directed to the drying system 1 (31). The liquid product resulting from this step is further directed to reactor 3 (23) in a counterflow system.

The thermo-induced drying system 1 (31) aims to dry the solid mass from the press 4 (26), in which drying occurs at temperatures below 70ºC, in continuous flow, where the material is exposed between 10 to 40 minutes. In a preferred embodiment, drying occurs at temperatures ranging from 35°C to 70°C. Variations in time and temperature may occur depending on the moisture conditions of the solid mass.

Optionally, the product obtained from the drying system 1 (31) can be subjected to a granulometry adjustment in a mill 2 (32) in order to meet commercial specifications.

In another embodiment, a second drying system 2 (34) may be necessary to obtain the dry solid mass.

After step 6.2, the functional prebiotic fiber containing polysaccharides (33') is obtained.

The functional prebiotic fiber obtained by the process of the present invention can be used in the food industry as a food, a food ingredient or food additive.

In view of the high capacity for water retention, water binding and emulsification, the functional prebiotic fiber of the present invention has application in bakery products, preparation of milk-based beverages, dairy foods, sauces and soups, processed meats, pet food.

The functional prebiotic fiber of the present invention also have properties that help control the pH of the skin, with application in cosmetic products.

The fractionation and total recovery process, object of the present invention, will be described below according to the particular, but nonlimiting, embodiments, since its embodiments can be carried out in different ways and variations and according to the application desired by the technician in the subject, with the scope of the invention being defined according to the claims.

A way of carrying out the process of the present invention compared to a way of carrying out a conventional process is shown below:

Initially, samples of orange citrus fruits of several varieties were collected and subjected to the following procedure:
I. The fruits were processed in an FTE -JBT Foodtech series juice extractor. The juice was weighed for mass balance calculations.
II. The bark in natura was collected and weighed.
III. The bark was processed in a tiger knife mill with a 32 mm screen, after which the same material was separated into two equal parts and sent to two types of process:
   - Example 1 - Conventional pectin extraction process; and
   - Example 2 - Total recovery process for citrus according to the present invention.

### EXAMPLE 1: CONVENTIONAL EXTRACTION PROCESS

Process No. 1 of conventional pectin extraction was conducted as follows: 18.0 kg of peel after standardization of granulometry was weighed and washed 3 times at a ratio of 2:1 with deionized water, until obtaining Brix 0, 8 in an open container using a shaker for 20 min.

Between each wash, the mixture was separated in a horizontal bagasse press to a final moisture content of 84-87%. The collected liquid was sent for analysis. Then, 15.0 kg of prepared bark, after final pressing, was added to a 50L pilot reactor, with a water/bark ratio of 2.5 : 1. The natural pH ranged from 4.2 to 4.5 , with temperature up to 65º C, for 60 min in constant agitation of 42 rpm. After 60 min the pH was adjusted to 2.0 with 65% nitric acid, adding 8-10% acid on dry basis. Feeding time was 240 min at 68°C.

After the extraction process, the liquid was filtered through a pilot vacuum filter.

The mass retained on the filter was weighed and dried at 65 (PECF) and the pectinous liquid was separated.

The pH of pectinous liquid was adjusted to 3.8 with 10% KOH solution and then directed to the ion exchange process for 30 min, making use of ion exchange resin.

The liquid was separated from the resin and precipitated in ethyl alcohol 92 GL. In the ratio of 2:1 for 3 consecutive times, the pectinous fiber being formed and pressed until the moisture reduction <60%BU.

The resulting sample was dried in an oven at a controlled temperature at 65ºC, until the sample moisture was reduced by 10% BS.

### EXAMPLE 2: METHOD OF CARRYING OUT THE INVENTION PROCESS

After processing the citrus peel in a knife tiger mill, part of the raw material is sent to process No. 2 containing the steps and parameters as proposed by the present invention.

Process No. 2 of total recovery of citrus fruits was conducted as follows: 15.0 kg of fresh peel after standardization of granulometry, without any type of previous treatment, was added to a 50-liter pilot reactor, receiving the mixture with the solvent hydrated ethyl alcohol in a proportion of 2:1 with a complexing solution of EDTA 15%, obtaining a mixture of 43 GL. The natural pH of the mixture was verified at 3.5-4.5 and stirring was maintained for 60 minutes.

After a time of 60 min, the pH of the mixture is adjusted with 65% nitric acid, to a pH of approximately 3.5. Less than 2% of acid solution was added to the mixture in relation to the dry base, stirring was maintained for 60 min.

The process is then directed to a mechanical horizontal bagasse press, where moisture reduction occurs. The liquid is recovered for the flavonoid fractionation process.

The solid mass returns to the reactor for a new mixture with hydrated ethyl alcohol in a ratio of 2:1 with a complexing solution of EDTA 15% and nitric acid 65%, with the pH adjusted to 3.5. The acid concentration in the mixture is less than 2% on a dry basis. Stirring was maintained for 60 min, obtaining a 43 GL mixture. The process is again directed to a mechanical horizontal bagasse press, where moisture reduction occurs. The liquid is recovered for the terpene fractionation process.

The solid mass returns to the reactor for a new mixture with hydrated ethyl alcohol in a ratio of 2:1 with a complexing solution of EDTA 15% and nitric acid 65%, pH adjusted to approximately 2.5. The acid concentration in the mixture is less than 2% on a dry basis. Agitation was maintained for 60 minutes, obtaining a 50 GL mixture. The process is then directed to a mechanical horizontal bagasse press, where moisture reduction occurs. The liquid is recovered for the counter flow process from sample A1.

The solid mass returns to the reactor for a mixture with hydrated ethyl alcohol in a 2:1 ratio, being stirred for 30 min until obtaining a mixture of 60 GL. The process is directed to a mechanical horizontal bagasse press, where moisture reduction occurs. The liquid is recovered for the counter flow process from sample A2.

The solid mass returns to the reactor for a mixture with hydrated ethyl alcohol in a 2:1 ratio, stirred for 30 min until a mixture of 70 GL, with pH adjusted with potassium hydroxide to 3.8. The process is directed to a mechanical horizontal bagasse press, where moisture reduction occurs. The liquid is recovered for the counter flow process from sample B.

The solid mass is directed to oven drying, returns to the reactor for a mixture with hydrated ethyl alcohol in a 2:1 ratio, for 30 min until a mixture of 70 GL. The pH was adjusted with potassium hydroxide to 3.8. The solid mass was dried in an oven at a controlled temperature at 65º C, until the sample moisture was reduced by 10% BS.

### EXAMPLE 3: METHOD OF PERFORMING THE INVENTION PROCESS

An alternative embodiment of carrying out process No. 2 is described below:
15.0 kg of fresh bark after standardization of granulometry, without any previous treatment, was added to a 50-liter pilot reactor, receiving the mixture with the solvent hydrated ethyl alcohol in the proportion of 2:1 with a complexing solution of EDTA 15%, obtaining a mixture of 43 GL. The natural pH of the mixture was found to be 3.5-4.5.

After a time of 60 min, the pH of the mixture is adjusted with 65% nitric acid, to a pH of approximately 3.5. Less than 2% of acid solution was added to the mixture in relation to the dry base, stirring was maintained for 60 min.

The process is then directed to a mechanical horizontal bagasse press, where moisture reduction occurs. The liquid is recovered for the flavonoid fractionation process.

The solid mass returns to the reactor with the liquid from the previous test (samples A2) in a 2:1 ratio with a complexing solution of EDTA 15% and nitric acid 65%, with the pH adjusted to 3.5. The acid concentration in the mixture is less than 2% on a dry basis. Stirring was maintained for 60 min, obtaining a 43 GL mixture. The process is again directed to a mechanical horizontal bagasse press, where moisture reduction occurs. The liquid is recovered for the terpene fractionation process.

The solid mass returns to the reactor with the liquid from the previous test (samples A1) in a 2:1 ratio with a complexing solution of 15% EDTA and 65% nitric acid, with pH adjusted to approximately 2.5. The acid concentration in the mixture is less than 2% on a dry basis. Agitation was maintained for 60 minutes, obtaining a 50 GL mixture. The process is then directed to a mechanical horizontal bagasse press, where moisture reduction occurs. The liquid is recovered for analysis of terpenes and flavonoids.

The solid mass returns to the reactor for a mixture with hydrated ethyl alcohol in a 2:1 ratio, being stirred for 30 min until obtaining a mixture of 60 GL. The process is directed to a mechanical horizontal bagasse press, where moisture reduction occurs. The liquid is recovered for analysis of terpenes and flavonoids.

The solid mass returns to the reactor for a mixture with hydrated ethyl alcohol in a 2:1 ratio, stirred for 30 min until a mixture of 70 GL, with pH adjusted with potassium hydroxide to 3.8. The process is directed to a mechanical horizontal bagasse press, where moisture reduction occurs. The liquid is recovered for analysis of terpenes and flavonoids.

The solid mass is directed to oven drying and returns to the reactor for a mixture with hydrated ethyl alcohol in a 2:1 ratio, for 30 min until a mixture of 70 GL. The pH was adjusted with potassium hydroxide to 3.8. The solid mass was dried in an oven at a controlled temperature at 65º C, until the sample moisture was reduced by 10% BS.

### TEST 1: COMPARATIVE TEST OF THE PRODUCT OBTAINED IN A CONVENTIONAL EXTRACTION PROCESS IN RELATION TO THE PRODUCT OBTAINED BY THE PROCESS OF THE PRESENT INVENTION

To evaluate the potential of the prebiotic fiber (33') generated in the final process of the present invention, tests were carried out in order to evaluate the stabilization of the fiber when applied to a milk sample.

The samples were extracted from the tests performed based on the procedures described in examples 1, 2 and 3. In total, 42 tests were performed, generating 912 samples.

The generated samples were named as follows:
- PEC - Pectin sample from example 1
- PEC 2 - Sample of residual solid mass from the pectin process of example 1;
- Process 1 - liquid samples from the first complexation step, containing flavonoids, from example 3;
- Process 2 - liquid samples from the second stage of terpene complexation, from example 3; and
- Prebiotic fiber - sample of the total solid mass of the process.

To verify the efficiency of the prebiotic fiber obtained by the invention in relation to the pectin used conventionally, the tests were aimed at evaluating the milk protein stabilization capacity of the prebiotic fiber of the invention compared to the stabilization obtained by the pectin.

The preparation of samples for the stabilization test comprised the following steps:
1. Initially, a 1.00% sample solution is prepared;
2. 700 ml of deionized water was added to the beaker;
3. Add 10g of the sample into the water using mixing with a high speed stirrer and mix for 5 minutes; and
4. All solution was transferred to a 1000 mL volumetric flask and made up to this volume.

For each test, a batch of 5L of solution in 20% of standardized skimmed milk powder was prepared. The solution was adjusted to pH 4.2 with citric acid and kept under constant stirring for 5 min. Then, the solution was quickly transferred to 300 ml centrifuge tubes. The amounts of water, sample solution and milk for each individual drink are specified in Table 1 below:

**TABLE 1: SAMPLE PREPARATION DATA TO PERFORM THE COMPARATIVE MILK PROTEIN STABILITY TEST:**

| **% concentration Sample** | **Sample solution (g)** | **Water (g)** | **Milk 20% (g)** | **Total weight (g)** |
|---|---|---|---|---|
| 0,000 | 0 | 100 | 100 | 200 |
| 0,100 | 20 | 80 | 100 | 200 |
| 0,125 | 25 | 75 | 100 | 200 |
| 0,150 | 30 | 70 | 100 | 200 |
| 0,175 | 35 | 65 | 100 | 200 |
| 0,200 | 40 | 60 | 100 | 200 |

Then, the tubes with the concentrations were placed in a water bath with vibration and magnetic stirring on a grid for 30 min at 55°C. They were then subjected to centrifugation (Thermo nunc centrifuge centrifuge force up to 7000g adapter 75003788¬¬) and the sediment at the bottom of the centrifuge cone was dried at 50º C, until a humidity of 10% and weighed to determine the % of sediments.

The % of sediments is an important parameter for determining the stability of milk. Due to the decrease in pH in the milk, the casein molecules are destabilized causing the milk to curdle, thus generating sediments. The aim of this test is, therefore, to note the balance of casein stabilization after adding samples at different concentrations. The greater the % of pectin sediments, the greater the degree of destabilization and the lower the efficiency of the stabilizing agent (pectin or the prebiotic fiber of the invention).

The detailed result for test No. 1 is described in Table 2 below.

**TABLE 3: RESULTS FOR TEST Nº1 CARRIED OUT TO EVALUATE THE % OF PECTIN SEDIMENTATION AND ITS PERFORMANCE IN TERMS OF STABILIZATION.**

| **PEC- pectin sample** | | | **PEC2 - sample of residual solid mass from the pectin process** | | | **Prebiotic Fiber - Sample of the total solid mass of the process.** | | |
|---|---|---|---|---|---|---|---|---|
| **Cod** | **Conc. Unit%** | **% Pectin sedimentation** | **Cod** | **Conc. Unit%** | **% Pectin sedimentation** | **Cod** | **Conc. Unit%** | **% Pectin sedimentation** |
| PEC1 | 0,000 | **8,7347** | PEC1 | 0,000 | **8,7347** | FC1 | 0,000 | **8,7347** |
| PEC2 | 0,000 | **8,6241** | PEC2 | 0,000 | **8,6241** | FC2 | 0,000 | **8,6241** |
| PEC3 | 0,075 | **14,1542** | PEC3 | 0,075 | **11,5021** | FC3 | 0,075 | **12,4557** |
| PEC4 | 0,075 | **14,1698** | PEC4 | 0,075 | **12,3105** | FC4 | 0,075 | **12,6111** |
| PEC5 | 0,100 | **17,2837** | PEC5 | 0,100 | **11,8141** | FC5 | 0,100 | **14,6264** |
| **Cod** | **Conc. Unit%** | **% Pectin sedimentation** | **Cod** | **Conc. Unit%** | **% Pectin sedimentation** | **Cod** | **Conc. Unit%** | **% Pectin sedimentation** |
| PEC6 | 0,100 | **17,8785** | PEC6 | 0,100 | **11,3814** | FC6 | 0,100 | **16,2573** |
| PEC7 | 0,125 | **7,6767** | PEC7 | 0,125 | **11,7076** | FC7 | 0,125 | **5,7672** |
| PEC8 | 0,125 | **7,4589** | PEC8 | 0,125 | **11,5872** | FC8 | 0,125 | **3,7295** |
| PEC9 | 0,150 | **5,2879** | PEC9 | 0,150 | **11,7643** | FC9 | 0,150 | **5,1057** |
| PEC10 | 0,150 | **6,0655** | PEC10 | 0,150 | **11,7501** | FC10 | 0,150 | **5,4602** |
| PEC11 | 0,175 | **5,2341** | PEC11 | 0,175 | **11,5374** | FC11 | 0,175 | **2,4765** |
| PEC12 | 0,175 | **5,4191** | PEC12 | 0,175 | **11,3814** | FC12 | 0,175 | **3,1543** |
| PEC13 | 0,200 | **4,7398** | PEC13 | 0,200 | **11,6297** | FC13 | 0,200 | **3,2439** |
| PEC14 | 0,200 | **4,4471** | PEC14 | 0,200 | **11,3744** | FC14 | 0,200 | **2,4308** |
| PEC15 | 0,250 | **4,5009** | PEC15 | 0,250 | **11,7715** | FC15 | 0,250 | **3,5227** |
| PEC16 | 0,250 | **4,3367** | PEC16 | 0,250 | **11,8992** | FC16 | 0,250 | **2,8450** |
| PEC17 | 0,300 | **4,0226** | PEC17 | 0,300 | **12,6367** | FC17 | 0,300 | **2,6614** |
| PEC18 | 0,300 | **4,4350** | PEC18 | 0,300 | **12,5232** | FC18 | 0,300 | **3,9699** |
| **Performance %: 23,69** | | | **Performance %: 73,30** | | | **Performance %: 97,98** | | |

All other tests nº 2 to 42 confirmed that the PEC sample, obtained via pectin extraction, is capable of providing yields in the range of 20.6 to 28.85%, with an average of 24.57%. The other tests nº 2 to 42 also confirmed that the probiotic fiber sample of the present invention provides yields of 97.98 to 98.98%, with an average of 98.47% in yield.

The data also showed surprising that the fiber sample of the present invention, even containing a pectin content four times lower than conventional pectin, presented an excellent milk stabilization performance, obtaining a polynomial curve 27% more efficient, according to data in Figure 2.

A sample obtained from the pectin extraction process (example 1) was also tested, referring to PEC 2 (sample of the residual solid mass of the pectin process), in which it was found that this residual sample does not show any stabilization results .

### TEST 2: CHARACTERIZATION OF THE PRODUCTS OBTAINED BY THE PROCESS OF THE PRESENT INVENTION

The tests carried out in examples 1, 2 and 3 had their products sent for testing for their proper characterization.

In these tests, the final prebiotic fiber product had the following aspects evaluated: Yield, Water-holding capacity (WHC), Light and color transmission and SAG (Strain-induced Alignment in a Gel), which is the ability to gel pectin linked with sugars.

The color determination method was done by a Minolta Chroma Meter CR-300 meter.

The procedure for the SAG method follows the procedure described by Cox and Higby (Food Inds., 16, 441 (1944)) and Joseph and Baier (Food Technol. 3, 18 (1949)) and consists of a modification of the IFT method 5-54 (Food Technology. Vol 13, 496-500ff (1959)).

The method of light transmission with 1% solution, heated, is determined by conventionally known methodologies, by means of a spectrophotometer.

The yield results compared, both the average and the individual data, proved 4.5 times more yields in the prebiotic fiber of the present invention, with the same efficiency in the applications demonstrated by the SAG test.

The results of WHC compared, through average data or individual data, proved 2 times more water absorption of the prebiotic fiber of the present invention compared to the conventional pectin sample. Synthetic fibers conventionally marketed present values ranging from 10-15 g/g. The prebiotic fiber of the invention presents 45 to 70% more absorption than conventional fibers available on the market.

The pectin described in example 1, because it is soluble in water, did not show absorption results.

The compared results of the light and color transmission test, through the average or individual data, proved 2 times more water absorption of the prebiotic fiber of the present invention in relation to the pectin sample. Synthetic fibers conventionally marketed present values ranging from 10-15 g/g. The probiotic fiber of the invention, in turn, presents 45 -70% more water absorption than market fibers.

In the SAG test, the prebiotic fiber of the invention showed results close to the average or individual pectin sample, even the fiber having a 4 times lower content of pectin present in its composition.

When the fiber is adjusted to a sample amount in order to contain a content of 50% of pectin present in the analysis, the results presented were 2 times greater.

When the fiber is adjusted to a sample amount in order to contain 75% of pectin present in the analysis, the results presented were greater than 270, presenting outside the reading scale.

Finally, it was found that the samples of the residual mass of the pectin extraction process, obtained by the conventional process, showed results below the reading range, being inefficient to gel the product.

Thus, it was surprisingly found that the probiotic fiber of the present invention is able to offer the same functionalities as pectin and also enrich it with a product rich in natural fibers, attributing probiotic functionality.

The characterization results of the products are expressed in Table 3.

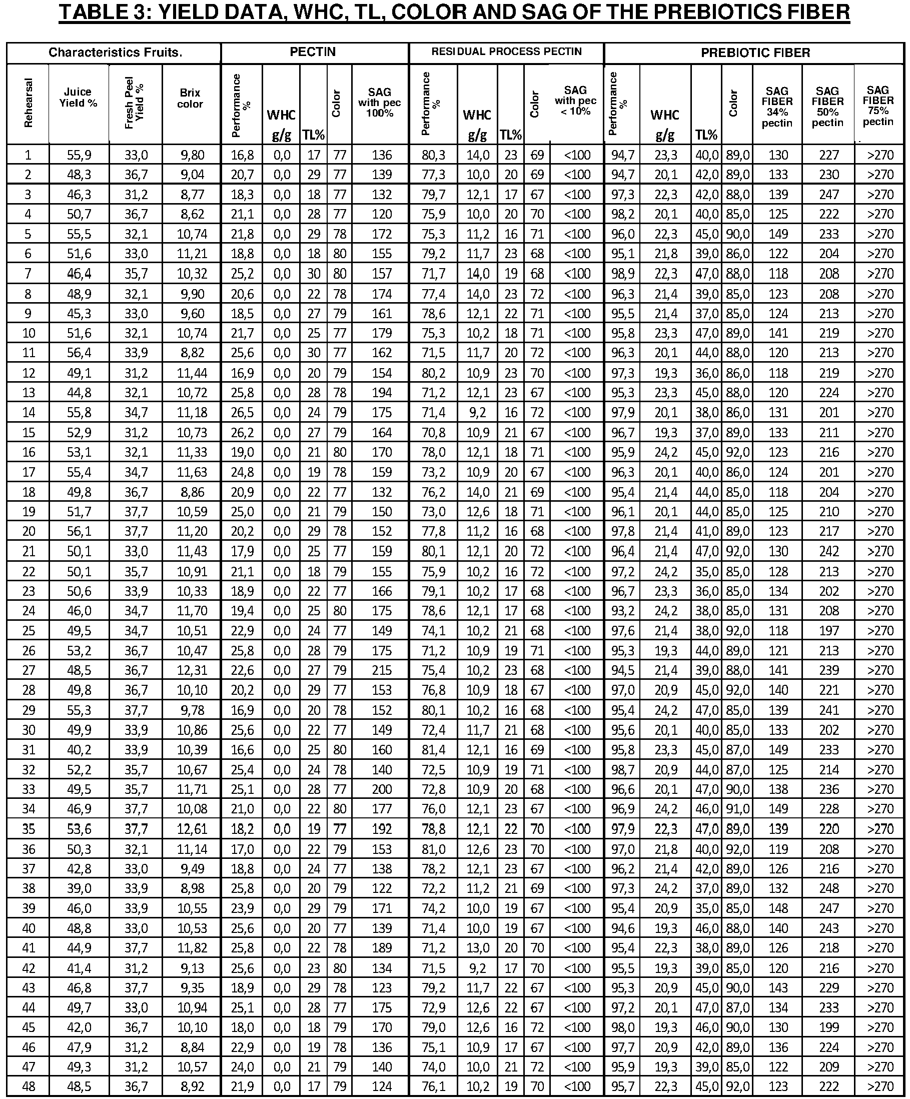

The characterization data of the prebiotic fiber of the present invention show WHC in the range ranging from 19.3 to 24.2 g/g, light transmittance ranging from 35 to 47 TL%, color ranging from 85 to 92 (which consists of a color whiter in relation to the commercially available yellowish color) and SAG from 118 to 149.

The other products generated in the process of the invention were also evaluated and characterized, as follows:
- Flavonoids: hesperidin diosmin consists of the type of flavonoids that are obtained through the conventional fruit method (known as chumbinho in its young phase) from citrus fruits after 2 weeks of petal fall.

The flavonoid is concentrated because it is not consumed because the fruit is small, but with the growth of the fruit, hesperidin is consumed by the fruit. When the juice is normally extracted, the pellets are also extracted in their young phase and, therefore, it is not possible for the fruit to grow and ripen, and consequently, it is not possible to extract the hesperidin diosmin.

From the method of the present invention, it is possible to recover hesperidin regardless of the stage in the fruit and the results have shown a yield of 0.3 to 2% in relation to the dry basis of the solid mass, with purity of 50 to 80 %.
- Citrus terpenes are recovered in conventional juice factory processes through evaporation and centrifugation, which are recovered from the water generated from the concentration of natural juice, where 60 - 70% of the oil available in the fruit is recovered.

However, between 25 to 40% of the terpene-rich oil is lost in the residual peel generated from the juice factory.

The process of the present invention allows the total recovery of the terpenes that were lost in the citrus peel, with a recovery of 100%, with the variation of yields depending on the citrus variety.
- The fertilizer is characterized by the minimum available NPK limits, with the nitrogen rate at 2%. In the process of the present invention, it was possible to reach rates of 2 to 6% of nitrogen without the need to add synthetic compounds to enhance its capacity.

## Claims

1. Process of fractionation and recovery of flavonoids, terpenes and polysaccharides from plant material, **characterized by** comprising the steps of:
1) Adjust the granulometry of the plant material (1') in a mill (1);
2) Place the ground plant material in a reactor 1 (3) and add a complexing compound (16) and a solvent (35);
3) Add an acidic compound (17) to the mixture obtained in step 2);
3.1- optionally, the vegetable water (8') that is recovered from the process, coming from the water tank 2 (15), is inserted into the mixture obtained in step 3);
3.2- separate the mixture obtained in step 3) to obtain a liquid phase containing flavonoids and terpenes and a first retained solid mass;
3.3- submit the liquid phase containing flavonoids and terpenes to a concentrator/distiller (7) to separate the flavonoids (13'), vegetable water (8') and NPK fertilizer (14') and the volatilized solvent (36), where said volatilized solvent returns to the process in reactor 4 (27);
4) Insert the first retained solid mass obtained in step 3.2 into a reactor 2 (19) and add the complexing compound (16), an acid compound (17), a solvent (35) and, optionally, at least one solvent recovered from the process (25 or 26);
4.1- separate the mixture obtained in step 4) to obtain a liquid phase containing terpenes and a second retained solid mass;
4.2 - submit the liquid phase containing terpenes to a concentrator/distillator (7) generating terpenes (10');
5) Insert the second retained solid mass obtained from step 4.1 into a reactor 3 (23) and add a solvent (35) and, optionally, at least one solvent recovered from the process (29 or 30);
5.1- separate the mixture obtained in step 5) to obtain a residual liquid phase and a third retained solid mass; and
6) Forward the third retained solid mass obtained in step 5.1 into a reactor 4 (27) and neutralize the mixture by adding a neutralizing compound (18) and the volatilized solvent (36) from the concentrator/distiller (7) and recover the fiber functional polysaccharide-containing (33').

2. Process, according to claim 1, wherein the plant material (1') is a peel of a citrus fruit, said fruit selected from orange, Tahiti lemon, Sicilian lemon, tangerine, lime, grapefruit, mandarin orange, pomelos and mixtures thereof and/or wherein the adjustment of granulometry of the plant material to occur in a mill (1) of knives, in a granulometry ranging from 2 to 20mm.

3. Process according to any one of claims 1 to 2, wherein the complexing compound (16) of step 2) and step 4) is selected from EDTA (ethylenediaminetetraacetic acid), DTPA (diethylenetriamine pentaacetic acid), NTA (nitrile triacetate) and mixtures thereof, in a concentration ranging from 5% to 40% by weight; and/or
wherein the solvent compound (35) of step 2) is selected from ethyl, propyl, butyl alcohol, or mixtures thereof, at a concentration ranging from 40% to 50 % by weight; and/or
wherein the mixture of plant material (1'), complexing compound (16) and solvent compound (35) from step 2) comprises a pH ranging from 3.0 to 4.5 and a temperature ranging from 20 to 35ºC.

4. Process, according to any one of claims 1 to 3, wherein the acid compound (17) of step 3) and step 4) is selected from: nitric acid, hydrochloric acid, sulfuric acid, acetic acid, or mixtures thereof; and/or
the concentration of the acid compound (17) entered in step 3) and step 4) is less than 5% by weight of the total volume of the mixture; and/or
the mixture of plant material (1'), complexing compound (16), solvent compound (35) and acid compound (17) from step 3) comprises a pH ranging from 2.5 to 3.5 and a temperature ranging from 20 to 35ºC.

5. Process according to any one of claims 1 to 4, wherein step 3.2 of separation of the mixture additionally comprises a sieve step 1 (5) and press 1 (6); and/or
wherein the sub-step 4.1 of separation of the mixture additionally comprises a sieve step 2 (21) and a press 2 (22).

6. Process, according to any one of claims 1 to 5, wherein the concentrator/distiller (7) of sub-step 3.3 includes heating the liquid phase obtained in sub-step 3.2 at a temperature ranging from 70 to 92ºC and concentration from 40 to 65º Brix; and/or wherein the concentrate containing flavonoids obtained in the concentrator / distiller (7) of sub-step 3.3 is optionally sent to a crystallizer (11), where a neutralizing compound (18) is added to a pH between 8 and 11, at a temperature ranging from 15 to 30ºC to crystallize the flavonoids (13'); and/or wherein the concentrate after crystallization (11) of the flavonoids obtained from sub-step 3.3 is subjected to tangential flow ultrafiltration (12) to separate the NPK organic fertilizer product (14').

7. Process, according to any one of claims 1 to 6, wherein the solvent (35) and at least one solvent compound recovered from the process (35, 25 or 26) of step 4) is selected from ethyl, propyl, butyl, or their mixtures, at a concentration ranging from 40% to 60% by weight.

8. Process according to any one of claims 1 to 7, wherein mixing a complexing compound (16), an acid compound (17), and at least one solvent recovered from the process (35), (25) or (26) from step 4) occur at a pH ranging from 2.5 to 4.5 and a temperature ranging from 35 to 55ºC.

9. Process, according to any one of claims 1 to 8, wherein the concentrator/distiller (7) of sub-step 4.2 includes heating the liquid phase containing terpenes obtained in step 4.1 at a temperature ranging from 40 to 95°C.

10. Process according to any one of claims 1 to 9, wherein at least one solvent compound recovered from process (35), (25) or (26) of step 5) is selected from ethyl, propyl, butyl, or mixtures thereof, at a concentration ranging from 40% to 60% by weight.

11. Process according to any one of claims 1 to 10, wherein the neutralizing compound (18) of step 6) is potassium hydroxide; and/or
wherein the neutralization step of step 6) occurs at a pH ranging from 3.5 to 6.5 and stirring from 30 to 42 rpm.

12. Process, according to any one of claims 1 to 11, wherein it additionally comprises a stage of dehydration of the solid mass by a drying system (31) thermo-induced at temperatures below 70°C.

13. Use of a plant material based on citrus peel to recover flavonoids, terpenes and polysaccharides, according to the process defined in any one of claims 1 to 12, wherein it is used to manufacture a food, cosmetic, pharmaceutical or chemical product.

14. Flavonoids, terpenes or prebiotic fiber containing polysaccharides, **characterized in that** it is obtained by a process as defined in any one of claims 1 to 12.

15. Prebiotic fiber containing polysaccharides, obtained by a process as defined in any one of claims 1 to 12, **characterized in that** it comprises WHC ranging from 19.3 to 24.2 g/g, light transmittance ranging from 35 to 47 TL%, color ranging from 85 to 92 and SAG from 118 to 149.
